# EUROPEAN PATENT SPECIFICATION

(11) **EP 2 701 587 B1**
(45) Date of publication and mention of the grant of the patent: **28.08.2019**
(21) Application number: 12715345.0
(22) Date of filing: 04.04.2012
(51) Int. Cl.: A61B 5/0408, B42D 3/18

(54) **ECG MAT**
EKG-MATTE
TAPIS POUR ÉLECTROCARDIOGRAMME

(30) Priority: 04.04.2011 GB 201105699
(43) Date of publication of application: 05.03.2014
(73) Proprietor: Cardiocity Limited, Colchester Essex CO2 7NN (GB)
(72) Inventor: CROCKFORD, Christopher John Dr, Warton Village Lancashire LA5 9QZ (GB)
(74) Representative: Slingsby Partners LLP
(86) International application number: PCT/EP2012/056243
(87) International publication number: WO 2012/136744

(56) References cited:
- WO-A2-2011/031062
- US-A1- 2002 045 805
- US-A1- 2008 069 375
- US-A1- 2010 292 589

## Description

### BACKGROUND OF THE INVENTION

The present invention relates to methods and apparatus for the acquisition and interpretation of ECG signals.

The use of electrocardiogram (ECG) detection and monitoring equipment has become pervasive, with ECGs being used to determine cardiac electrical activity of both animals and humans. ECG equipment is used for a variety of purposes: from measuring the cardiac performance of athletes, to observing basic heart function, through to the detailed monitoring of problems in specific sections of the cardiac system.

Long term ECG monitoring is an established practice, and the detection of the heart's rhythm when monitored over time can identify performance problems known as arrhythmias that can be classified according to the area of the cardiac system that has caused the rhythm to be disrupted. Short term monitoring can provide a means to understand momentary cardiac performance but will not provide a full insight into what may subsequently happen to the cardiac system.

Conventional ECG equipment monitors the electrical activity at the skin of a subject due to the beating of the heart and expresses the electrical activity as a waveform (an electrocardiogram). The electrical activity is detected by electrodes (typically Ag/AgCI electrodes) attached at specific points on a subject's anatomy and electrically coupled to the skin by a conductive gel. Modern ECG systems can make use of automated algorithms that analyse the structure, timing, and electrical characteristics of the cardiac systems various components.

The placement of the electrodes is conventionally determined in accordance with Einthoven's Triangular law, which allocates three electrodes as limb leads: Left Hand (LH), Right Hand (RH), and Left Foot (LF). The potential difference (typically millivolts) between LH and RH, being known as Lead 1, between LH and LF as Lead 2, and RH and LF as Lead 3. The term lead is used to refer to a conceptual investigative lead and not a physical electrode cable lead. By taking readings from the three limb leads it is possible to understand the electrical activity of the cardiac system from three angular perspectives. If the average of any two leads is then further compared against the third lead, it is possible to derive a further three investigative leads, known as the derived Leads 4-6.

A 6 lead systems allows an investigator to see a cardiac system's electrical activity from the centre of the source of the electrical signal along six different axes. To understand the effect of the electrical signal on specific muscle tissues of the cardiac system, further investigative leads (chest leads) must be attached to the body to look at the electrical signal passing through the regions of the heart. The deployment of chest leads (known as V1 through V6) in an arc from approximately the sternum to under the left hand side of the rib cage allows such a detailed reading of the electrical activity passing sequentially through the muscles of the heart. This allows various diseases, conditions and any damage to the heart to be determined.

Variations on the placement of leads has led to more derived leads being obtained through fewer electrodes, and various algorithms have been established to enable fewer electrodes to yield more information about the electrical activity of the cardiac system.

The algorithms used to identify different rhythm problems, or arrhythmias, use time based analysis of the outputs from the electrodes and care must be used to ensure that the correct time base is used when comparing one electrode's signal with another. The time stamping of individual signals can require a highly accurate and correlated time source.

The placement of conventional electrodes on the subject often causes issues, especially if the subject is hairy or has a skin condition that causes the subject's skin to react to the adhesive pad. Conductive gels can also cause irritation to the subject and also introduce the problem that the gel can provide low resistance paths between adjacent electrodes. The usage of conventional dry electrodes is limited since their signal to noise ratio is often very high.

Recently, "contactless" electrodes have been introduced that measure electrical potential at the skin without a direct electrical contact by means of capacitive coupling. These electrodes, commonly referred to as electric potential sensors, work by sensing the electric field created by displacement currents in the body of the subject. These electric potential sensors are discussed in International Patent Application Publication No. WO 01/16607, which describes an electric field sensor having a capacitive pick-up electrode for the detection of alternating electrical fields originating from within the human body. The electrode is connected to a high impedance sensing amplifier. In order to render the capacitance coupling relatively sensitive to variations in the separation between the body and the electrode, the electrode itself is separated from the body by a thin (preferably low dielectric) insulating layer, and a limiting capacitor is placed in series with the input to the sensing amplifier. Thus the sensor can be seen to be contactless.

Further relevant publications include European Patent No. 1451595, Japanese Patent Application NO. 2005511174, Taiwanese Patent Application No. 2003200664, US Patent Application No. 20060058694, and International Patent Application Publication No. WO 03/048789. These describe the operation of an electro-dynamic sensor, in which a number of different circuit techniques are combined to achieve several orders of magnitude improvement in sensitivity compared to previously known electro-dynamic sensors, whilst still maintaining sufficient stability to permit a measurement to be acquired in everyday conditions.

These publications describe an electrodynamic sensor comprising a high input impedance electrometer adapted to measure small electrical potentials originating from the subject under test and which employs at least one input probe having no direct electrical contact with the subject. In order to function, the circuit arrangement of the electrometer comprises an amplifier that includes a combination of ancillary circuits providing feedback from the output of the amplifier and arranged cumulatively to increase the sensitivity of the electrometer to the small electrical potentials whilst not affecting the electrical field being monitored. Such ancillary circuits typically provide at least two characteristics that can refine the signal, such as guarding, bootstrapping, neutralisation, supply rail drift correction, supply modulation and offset correction for the sensor.

However, the use of the output signal from the amplifier as the feedback signal has the disadvantage that such a signal is a broadband signal, which may have a poor signal to noise ratio. This noise is then fed back to the amplifier input by the feedback setup, causing further degradation of the signal to noise ratio.

In contrast, the feedback signal of an electric potential sensor is not a broadband signal derived directly from the output of the sensor, but is a generally a coherent signal. This has the effect of significantly improving the signal to noise ratio and makes electric potential sensors a better alternative to conventional wet electrodes for ECGs than electrodynamic sensors.

The fact that EP sensors allow capacitively coupled measurements to be taken through thin clothing, without the use of conductive gels and whilst maintaining good electrical isolation from the subject means that this new class of sensors is very promising for use as an alternative to conventional dry ECG electrodes. However, the use of electric potential (EP) sensors using capacitance coupling for data acquisition is not without its own inherent problems, including: how to best couple the sensors to a subject, how to isolate the highly sensitive sensors from external interference, and how to best process the outputs of one or more EP sensors in order to effect the analysis of a detected ECG. These problems are addressed by the inventions described herein.

US 2012/172729 describes an apparatus for measuring biometric information having a hand rest. The apparatus for measuring the biometric information having the hand rest includes a hand rest formed in a concave curvature shape to have an examinee's hand put thereon, to contact with an examinee's palm and fingers; and a measurement part provided in the hand rest to measure the examinee's biometric information.

US 2010/292589 describes a device having an arterial pulse sensor adhered to the hypothenar region of the palm using an adhesive patch. The patch has an adhesive surface that is covered by a removable film with an outer portion and a central portion.

US 2008/0069375 describes a setup, according to the preamble of claim 1, where a differential ECG signal is acquired from two fingers, one of each hand, using dry electrode plates. Motion artifacts cancellation is implemented by local surface noise recording. The contact sensor plates are divided into two reception zones to allow for both a local surface noise recording from the left and right fingers, as well as for recording of the differential ECG signal.

### SUMMARY OF THE INVENTION

According to a first aspect of the present invention as defined in claim 1 there is provided an electrocardiographic device for sensing cardiac activity in a user comprising: a pair of electrically insulating user interaction regions, each adapted for engagement with a hand of a user; a first set of electric potential sensors provided at the user interaction regions and each configured to provide an electric potential sensor output signal; a first set of indicia indicating a first position of a user's hands with respect to the first set of electric potential sensors; and at each user interaction region, a movement sensor configured to provide a movement signal that, in use when a user's hands are in the first position, represents movement and/or vibration of the user's hands; wherein the first set of sensors are arranged relative to the first set of indicia such that, in use when a user's hands are in the first position, an electric potential sensor is located under the thenar eminence and/or an electric potential sensor is located under the hypothenar eminence of each of the user's hands; and wherein the electrocardiographic device is configured to use each movement signal to filter out corresponding variations in the electric potential sensor output signal(s) from the electric potential sensors of the respective user interaction region.

Suitably the indicia comprise a graphical pair of hands indicating the desired location of a user's hands relative to the sensors and/or indentations on the surface of the device indicating the desired location of a user's hands relative to the sensors.

Preferably each user interaction region comprises a layer of shape memory foam for engagement with a user's hand, the first set of electric potential sensors being provided at the shape memory foam.

Preferably each user interaction region is supported on a floating plate elastically coupled to the bulk of the electrocardiographic device. Both user interaction regions could be supported at the same floating plate. Each floating plate could be elastically coupled to the bulk of the electrocardiographic device by one or more of: a spring optionally with a damper, a rubber or plastic element, and elastic foam.

The movement sensing means could be one or more of an accelerometer, a strain gauge, a microphone, a pressure sensor, and an EP sensor arranged such that, in use, the EP sensor does not engage with the user.

The electrocardiographic device could further comprise a second set of indicia indicating a second position of a user's hands with respect to the first set of electric potential sensors; wherein the first set of sensors are arranged relative to the second set of indicia such that, in use when a user's hands are in the second position, at least one sensor is located under the medial side of the hypothenar eminence of each of the user's hands.

Suitably the electrocardiographic device comprises at least two electric potential sensors at each user interaction region.

Preferably each electric potential sensor comprises a conducting electrode coated with a thin insulating layer such that, in use, at least the thin insulating layer separates the user from the conducting electrode.

Preferably each electric potential sensor is provided within but electrically insulated from a conductive patch such that, in use, the user is in electrical contact with the conductive patch. Preferably the conductive patch is connected to an electrical ground of the electrocardiographic device.

The electrocardiographic device could further comprise a display screen operable to display one or more of: a graphic illustrating a user's hand engaging with a user interaction region in the first position; a message directing the user to engage with a user interaction region in a position other than the first position; an electrocardiograph acquired by the first set of electric potential sensors.

Suitably the electrocardiographic device is configured to be foldable along one or more folds such that, when folded, the device is operable to present at least one of the first set of electric potential sensors on each of two outward-facing surfaces of the device, the two outward-facing surfaces being separated by at least one fold.

Suitably the electrocardiographic device is provided in the form of a book having one or more pages, at least one of the first set of electric potential sensors being provided at the front and rear inside covers of the book. The pages of the book could provide instructions for use of the electrocardiographic device.

### DESCRIPTION OF THE DRAWINGS

The present invention will now be described by way of example with reference to the accompanying drawings, in which:
Figure 1 is a schematic diagram of an electrocardiographic device for sensing cardiac activity in a user in accordance with the present invention.
Figure 2 is a schematic diagram of a foldable ECG mat configured in accordance with the present invention.
Figure 3 illustrates schematic diagrams of two preferred embodiments of an ECG mat in accordance with the present invention.
Figure 4 is a schematic diagram of an ECG mat configure in accordance with the present invention and having multiple sensors at each user interaction region.
Figure 5 is a schematic diagram of an ECG mat configured in accordance with the present invention and provided in the form of a book.

### DETAILED DESCRIPTION OF THE DRAWINGS

The following description is presented to enable any person skilled in the art to make and use the invention, and is provided in the context of a particular application. Various modifications to the disclosed embodiments will be readily apparent to those skilled in the art.

The present invention is not intended to be limited to the embodiments shown, but is to be accorded the widest scope consistent with the principles and features disclosed herein.

Due to the low sensitivity of conventional electrodes and the nature of the electrical signals generated by cardiac activity, it has been necessary to accurately locate conventional ECG electrodes. The present invention recognizes that the spatial location of electric potential sensors can challenge the conventional locations for applications in which detailed monitoring of the electrical activity in the heart is not required (such applications are generally handled by 12 lead ECG machines). The present invention provides the performance of conventional 1 to 6 lead ECG machines, depending on the configuration of a device configured in accordance with the present invention.

The present invention further recognizes that the variation in an acquired electrocardiograph due to variations in the position of the EP sensors on the user's body has several novel uses.

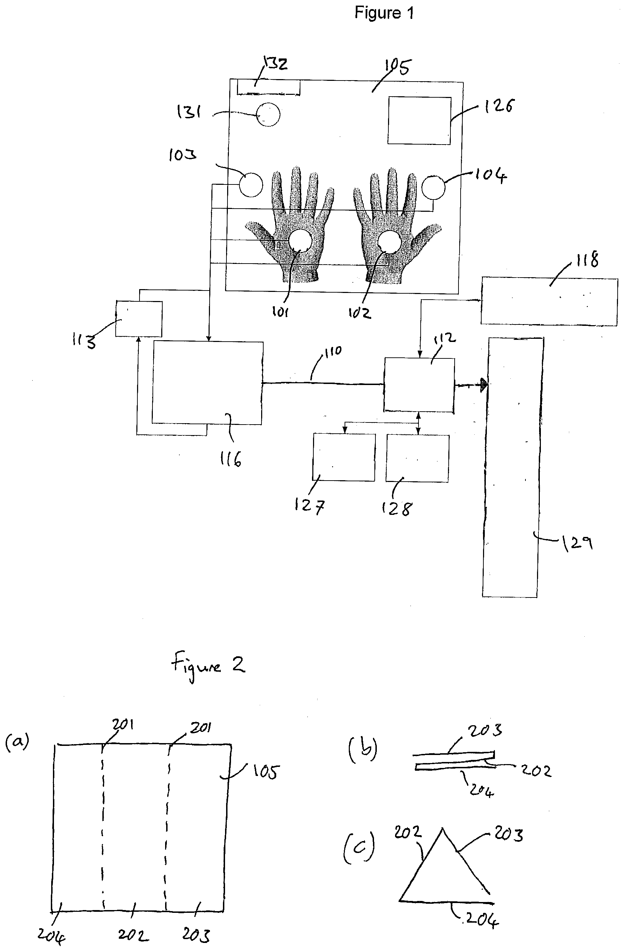

A schematic diagram of an electrocardiographic system comprising an electrocardiographic device for sensing cardiac activity in a user is shown in figure 1. The two hands are shown by way of illustration only and are an example of one use of the electrocardiographic device. The electrocardiographic device includes EP sensors 101 and 102, and optionally EP sensors 103 and 104. EP sensors 101 and 102 are set into an insulating mat 105 which includes one or more sets of indicia indicating where on the mat the user is intended to place their hands and/or feet (the case of hands is illustrated in figure 1). A mat could comprise sets of indicia for both hands and feet so as to allow a mat to be used to acquire ECG signals from either hands or feet. These indicia may be in the form of outline shapes showing where the hands/feet should be placed, and/or indentations in the surface of the mat showing where the hands/feet should be placed. Most advantageously, the mat is configured such that: (a) in the case of hands: the hands of a user are placed so that at least sensors 101 and 102 lie under the ball of the thumb of each hand when in use, with the palms face-down on the mat; and (b) in the case of feet: the feet of a user are placed so that at least sensors 103 and 104 lie under the ball of each foot when in use, with the feet soles-down on the mat. This helps to shield the sensors from environmental noise.

Each sensor feeds its data to an analogue to digital converter 116, with each sensor typically being on a different channel. The analogue to digital convertor may incorporate a feedback loop 113 for controlling the signal to noise ratio and the background environmental signals being amplified. Preferably the AtoD and feedback loop includes an amplifier for coherent amplification of EGC signals received from the EP sensors. Preferably data representing the output from the sensors is passed to a control unit 112 over link 110, which is typically a microcontroller or microprocessor.

The control unit or data processing system could be located at one or more of the electrocardiographic device, at an external data processing device (such as a laptop or smartphone), or at a remote data processing device (such as an internet server) accessible to the electrocardiographic device. Thus different aspects of the filtering, signal processing and electrocardiogram analysis could be performed at different parts of a data processing system formed by processing elements at one or more of the electrocardiographic device, computer or cloud server. In preferred embodiments, signal filtering and conditioning is performed at the electrocardiographic device and electrocardiogram analysis is performed at a connected computer, with the option of uploading data to a server for more rigorous analysis should the need arise. Link 110 could be any kind of wired or wireless connection, such as a serial bus, Ethernet link or Bluetooth connection.

Data processing system/control unit 112 is configured to process the data representing the output from the sensors in the manner required by the application. Preferably the control unit is operable to process the data in accordance with a set of algorithms stored at data store 118 (preferably local to the control unit) for identifying arrhythmias. The output 129 from the control unit may be in the form of a set of one or more rhythm strips of ECG data. The output could be stored at the device and/or displayed at display screen 126. If the control unit is local to the device the output could be transmitted over optional wired or wireless interfaces 127 or 128. The screen may be integral to mat 105 or could be provided separately. Optional wireless interface 127 may provide telephonic interfaces such as GSM or 3G. Optional wireless interface 128 may provide other radio interfaces such as Bluetooth or WiFi. The control unit may alternatively or additionally output a statistical analysis of the ECG rhythm strips, indications of any cardiac abnormalities detected by its processing, and instructions to the user.

The sensors 101 to 104 being EP sensors need not be in direct contact with the skin of a subject in order to sense an ECG signal. For example, a user may engage with the EP sensors through one or more layers of clothing. That part of the EP sensors with which a body part is engaged could be enclosed within the material of the mat such that engagement of a user with the EP sensors occurs through the insulating material of the mat. Such an arrangement can help to minimise the level of unwanted environmental noise leaking into the EP sensors. Suitable sensors include the EPIC Ultra High Impedance ECG Sensors manufactured by Plessey Semiconductors, such as the PS25201A.

In the example shown in figure 1, a mat or pad is provided onto which the subject under investigation can arrange its hands upon sensors 101 and 102 in order to allow a single Lead I ECG reading to be recorded as the potential difference between EP sensors 101 and 102. The mat or pad 105 could be provided with additional sensors 103, 104 in order to capture ECG readings from additional body parts - these sensors could be integral with the mat or connected to the mat by wired or wireless connections. EP sensors 103, 104 could be provided in clothing, devices adapted for attachment to a patient (such as a velcro strap comprising one or more EP sensors for connection around a wrist or ankle), or a chest harness comprising a plurality of EP sensors, to give some examples. The mat could be configured so as to capture readings from all sensors simultaneously. The provision of multiple sets of sensors allows a mat to be used in multiple configurations - for example, to take 1-lead readings from either a pair of hands, a pair of feet, or a foot and a hand, or to take multiple lead readings from a combination of hands, feet or other parts of the body.

Additional EP sensors could be provided by another mat configured in accordance with the present invention, the two mats being connected together so as to allow the differential processing of pairs of EP sensors. The mats could be connected together at a control unit such as a laptop. For example, a user could stand on one mat and engage one or more feet with feet sensors 103 and 104, whilst engaging their hands with sensors 101 and 102 of a second mat. Both mats would be coupled to control unit 112. This allows ECG leads 1 to 3 to be obtained, and hence the derivation of leads 4 to 6.

EP sensors configured in accordance with the present invention can be located at the limbs of a subject so as to capture multi-lead ECG signals in an equivalent manner to conventional multi-lead ECG systems having electrodes located at the same positions, but with the benefit that no adhesives or conductive gels need to be used.

In the particular example of a mat having sensors configured to engage with a user's feet, it should be noted that the use of EP sensors allows the mat to pick up ECG signals through the thick skin of the soles of the user's feet, and possibly also through a layer of clothing, such as socks. The provision of a mat comprising EP sensors conveniently allows the ECG signal of a user to be captured simply by stepping onto a mat configured in accordance with the present invention, and optionally placing their hands on another mat.

It is particularly advantageous that the parts of the user's anatomy that are placed on the mat are arranged such that the user's anatomy shields the EP sensors from environmental noise. This can improve the fidelity and speed of detection of an ECG system that uses EP sensors. For example, the positioning of the hand such that the ball of the thumb of the hand is located over an EP sensor provides a good ECG signal and allows the thick, fleshy part of the hand to shield the EP sensor from environmental noise. This speeds up ECG acquisition time by the EP sensor and lowers noise levels present in the signal. The positioning of a foot such that the ball of the foot (or base of the big toe) lies over an EP sensor is similarly advantageous and it is preferably if a mat adapted for use with a user's feet is provided with indicia such that in use the user is directed to engage with the EP sensors in such an advantageous manner.

It is advantageous if a feedback system is provided for use with the mat so as to ensure that the limbs of a user are placed in their optimal positions in order to best shield the EP sensors from environmental noise. When ECG signals are acquired by the mat but those signals are of poor quality, the control unit 112 is preferably configured to alert the user to the fact that their limbs may not be optimally placed on the mat and/or to indicate to the user to add one or more additional EP sensors so as to provide additional leads and/or to indicate to the user to engage with different EP sensors of the mat. The control unit can determine whether a signal is poor quality by comparing an acquired ECG signal with sets of characteristics stored at data store 118, at least some of which indicate that there is poor engagement between the user and the respective electric potential sensors

As is known in the art, the mat is configured to use a QRS detection algorithm to identify the heart beats in the signal from the EP sensors. This allows the amplitude of the QRS complex and the timing between features of the ECG to be established, along with measures of the noise in the signal. Further refinements can also be employed to improve the quality of the signal, such as filtering, use of a baseline wander algorithm to better track the varying signal and use of cubic spline operations to stabilise the ECG.

Various aspects of the ECG of a user can be used to both characterise the ECG and hence heart function, and allow the quality of the captured ECG to be determined. Characterisation of the ECG allows the ECG to be stored as a set of parameters describing the ECG and can be used for later analysis or for comparison against subsequently-captured ECGs. Suitable aspects of the ECG that can be stored for comparison or for determining quality include:
1. Amplitude and timing of the QRS complex (this could include, absolute amplitude of the R peak, absolute or relative amplitudes of the Q and S peaks, duration of the QRS complex, ventricular activation time, absolute or relative timings of the Q, R and S peaks);
2. R-R interval (i.e. the user's pulse);
3. QT complex (this represents the electrical depolarization and repolarization of the left and right ventricles);
4. P wave timing relative to the QRS complex (this is most easily identified from Lead II, which can be captured from the differential signal from an EP sensor at the right hand and an EP sensor at the left foot;
5. T wave to P wave smoothness (the presence of bumps - i.e. a low smoothness - can indicate atrial fibrillation, although this diagnosis is best confirmed by taking a multiple lead ECG).

A low quality signal can be indicated if, amongst other things, the QRS complex cannot be identified, if its amplitude is small, if significant variation is observed in any of the above characteristics that is unlikely to be due to the cardiac activity of the user, or if a significant noise level is observed in the signal.

Discussions of signal filtering and quality determination suitable for use in the systems described herein are set out in the following US patent applications, serial numbers 11/470,506; 12/624,175; and 12/424,046.

Preferably the control unit directs the user to adjust the placement of their limbs (e.g. hands or feet) on the mat and then determines whether the signal quality has improved. The control unit may direct the user to make multiple adjustments and then ask the user to adopt the position that was associated with the best signal quality. The control unit may be configured to present such directions to the user by means of screen 126, which may or may not form part of the mat 105.

It is advantageous if shape memory foam whether low density or high, provides the surface of mat 105 in the region in which the hands or feet are configured to engage with the EP sensors. This allows the mat to mould to the shape of the user's hands or feet and helps to reduce tremors and the effect of signal noise due to the vibration or movement of the hands or feet. The support provided by memory foam is of particular importance for users with certain conditions, such as rheumatism or certain neurological conditions. Preferably the shapes of hands or feet are initially imprinted into high density memory foam to provide a guide as to how users should place their hands on the mat. Alternatively low density foam may be used to mould the foam around the users hands and thus provide a temporal support. Such hand- shaped or feet-shaped depressions in the memory foam could serve as the sets of indicia discussed above that indicate where on the mat the user is intended to place their hands or feet. The EP sensors 101, 102 are preferably embedded in the memory foam with the sensor surface exposed for engagement with the user. The sensor surface is preferably level with the surface of the memory foam or slightly below the surface of the memory foam such that when the foam is compressed the hand/foot engages with the sensor. A suitable memory foam is Veriltye (RTM), manufactured by the Cornerstone Research Group, Inc.

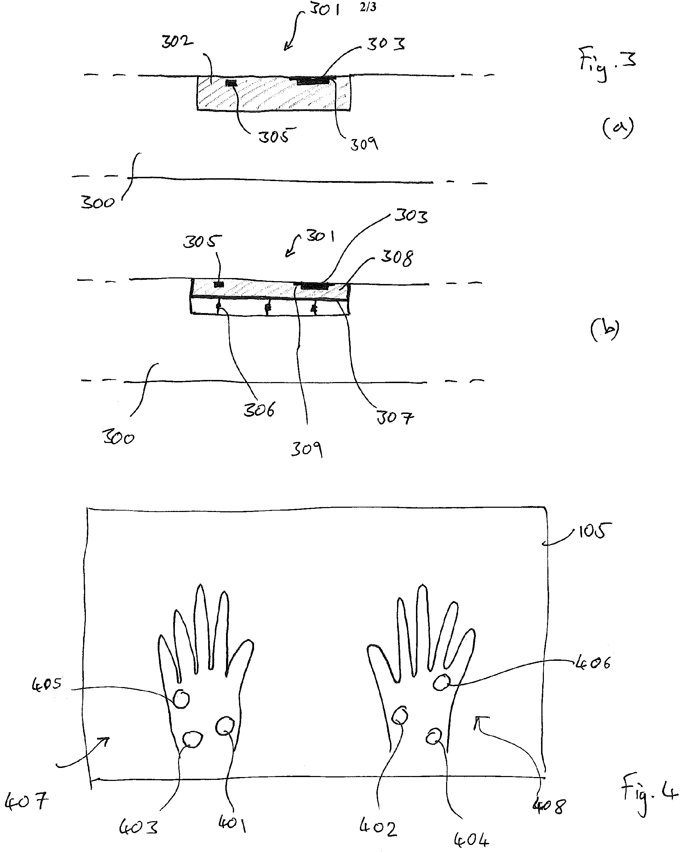

It is further advantageous if a conductive region is arranged around each EP sensor such that the conductive region is in contact with the user when the user is engaged with the EP sensor. The conductive region is preferably grounded relative to the signal processing electronics of the mat and could be earthed if the mat is itself earthed. The conductive region is preferably a metal, such as copper. The conductive region could be in the form of a conductive ring with the EP sensor at the centre of the ring, as shown in figures 3 (a) and (b). The conductive region could form at least part of the indicia arranged to indicate to the user how they are to engage with the mat.

The device optionally includes a camera 131. The output from the camera can be fed to the control unit so as to allow the control unit to interactively instruct the user as to the optimal placement of their hands. An optional mirror 132 may also be provided that can be configured to increase the camera's field of view.

The use of a camera 131 allows the position of the user's limbs on the mat relative to the EP sensors to be monitored. The output of the camera is fed to control unit 112 which can be configured to direct the user to place their limbs in the optimal position. This may be in response to acquiring a poor quality signal. For example, the control unit may monitor by means of the camera the position of a user's hands on the mat. If the user's hands are not in the optimal position, the control unit could direct the user to improve their position by means of images or text on screen 126 that identifies to the user how their position might be improved. The camera could alternatively or additionally be used to identify movement of the user's hands such as tremors at the time of data acquisition. Artefacts in the signals from the EP sensors that correspond to movement observed by the camera can be filtered from the signal using filtering algorithms well known in the art.

The provision of a camera 131 may alternatively allow the control unit to adapt its processing of the acquired ECG signals in dependence on the position of the user's limbs as detected by the camera relative to the EP sensors. A mirror 132 may also be provided at the mat so as to allow the camera to identify the position of a user's limbs at points that are not visible by direct line of sight from the camera aperture. Alternatively or additionally the camera could have multiple apertures and sensors, or there could be multiple cameras 131.

A preferred embodiment of the invention is shown in figure 3. It is advantageous if the user interaction region 301 of the surface of the mat 300 with which the user engages their hands or feet is provided with one or more accelerometers 305 configured to detect the movements and vibration of the user's hands/feet. Most preferably the user interaction region of the surface of the mat is decoupled from the rigid body of the mat. For example, in figure 3(a) the user interaction region of the surface of the mat comprises a compressible foam 302 in which the EP sensors are supported (such as the memory foam described above), or in figure 3(b) the user interaction region is a floating plate 307 decoupled from the rigid body of the mat by means of one or more springs, elastic compressible materials such as rubber, or any other kind of resilient couplings 306.

In the case in which the user interaction region is a floating plate, the EP sensors 303 would be supported at the floating plate. The floating plate could itself support a compressible foam 308, such as a memory foam. The one or more accelerometers 305 could be located at the floating plate or could be configured to measure motion of the floating plate relative to the rigid body of the mat - for example, by means of one or more strain gauges connected between the mat and floating plate. If a compressible foam is used in the user interaction region of the surface of the mat, whether the foam is atop a floating plate or not, the accelerometers 305 would preferably be embedded in the portion of the compressible foam close to the surface with which the user's hands or feet engage. Accelerometers 305 could be any kind of accelerometer, such as a tilt sensor, piezoelectric accelerometer, capacitive accelerometer, or micro electro-mechanical (MEMS) device.

Preferably each user interaction region comprises at least one accelerometer. Most preferably the user interaction regions of a mat are decoupled from one another in that each region is a distinct region of compressible foam supporting one or more EP sensors, or each surface region is independently supported at a floating plate. Figures 3 (a) and (b) each show a single user interaction region, thus EP sensor 303 might correspond to sensor 101 of figure 1 with region 301 corresponding roughly to the area indicated by one of the hands in figure 1. Typically each user interaction region corresponds to a particular point of interaction with the user, such as a hand or a foot.

Vibrations and movement of the user introduces noise into the electric potential signal detected by sensors 303. By providing accelerometers 305, the effect of this noise on the EP signal can be mitigated and the quality of signal improved. The output of accelerometer 305 provides a measure of the vibration and movement of the respective body part of the user that is engaged with the user interaction region 301. The accelerometer output can therefore be used to filter out corresponding variations in the signal from EP sensor 303 that are likely to be due to noise induced by the vibrations and movement of the user. Various algorithms could be used to achieve this. For example, when significant movement is detected by an accelerometer (or by the camera, as described above), variations in the signal from the corresponding EP sensor could be suppressed; and any periodic variations above a predetermined frequency (preferably around 30-100Hz) present in both the output from the accelerometer and the EP sensor signal could be filtered from the EP sensor signal. This is particularly important in order to allow the system to identify uneven artefacts in the T-P wave part of the ECG that are suggestive of atrial fibrillation. Filtering algorithms for removing or suppressing features in a first signal (the ECG) that correlate with features in a second signal (the accelerometer output) are well known in the art.

It can be advantageous to use EP sensors of the mat with which the user is not engaged (e.g. sensors 103 and 104 of figure 1) to measure movement or vibration of the user. Since the user is not engaged with those sensors, they will not pick up the ECG of the user. However, the ECG sensors will to some extent act like microphones and this signal can be used in the filtering algorithms to remove or suppress the effect of vibrations from the ECG signal. In alternative embodiments, dedicated EP sensors or one or more microphones could be provided for this purpose instead or in addition to one or more accelerometers.

The output of one or more accelerometers, microphones or cameras configured to detect movement of the user could be stored with captured ECG data for later analysis. Data captured at the mat could be uploaded to an internet server (i.e. the "cloud") for more detailed analysis at more powerful servers or to allow a cardiologist to review the ECG trace - this could be requested by the patient or healthcare professional at the mat or control unit.

Some users may suffer from conditions that make it difficult to place their hands/feet in the intended manner on the user interaction regions of the mat. For example, users with advanced rheumatoid arthritis may find it difficult to lay their hands even roughly flat on the mat such that the EP sensors lie directly under the balls of their thumbs. For such users it is preferred that the mat is configured to have indicia that indicate to the user a secondary hand position (not shown in figure 1) in which the hypothenar eminence (the fleshy part of the medial side of the hand) is located over EP sensors 101 and 102. A second set of indicia need not be required if, as shown in figure 4, the mat provides multiple sensors per hand and the mat provides a mode in which it uses only those EP sensors under the hypothenar eminence when the user places their hands as best they can on the mat. Such a mode would preferably be selectable by the user or a healthcare professional to indicate to the mat that the user cannot place their hand in the optimal, primary hand position.

In order to improve the quality of the ECG signal captured for such users, it is advantageous to provide a plurality of EP sensors for engagement with the user at each user interaction region - i.e. a plurality of EP sensors is provided for engagement with each hand or foot. By choosing the pair of outputs from a pair of user interaction regions that provide the cleanest signal, a mat configured according to a preferred embodiment of the present invention can maintain acceptable signal quality for a wide range of orientations of the user's hands and/or feet.

In order to select the optimum pair of EP sensor outputs, the differential signals can be calculated for each of the possible combinations of a plurality of EP sensors at a first user interaction region with a plurality of EP sensors at a second user interaction region. The quality of those signals can then be established by means of a QRS detection algorithm as described above. For example, with reference to figure 4, each of the outputs from EP sensors 401, 403 and 405 at the first user interaction region 407 is paired with each of the outputs from EP sensors 402, 404 and 406 at the second user interaction region 408 so as to form a set of 9 differential outputs d₁ to d₉ as set out in Table 1 below.

| EP Sensor | 402 | 404 | 406 |
|---|---|---|---|
| 401 | d₁ | d₂ | d₃ |
| 403 | d₄ | d₅ | d₆ |
| 405 | d₇ | d₈ | d₉ |

In some embodiments, only a subset of the possible differential outputs could be calculated so as to generate, for example, only those differential outputs that have been found to provide acceptable quality ECG signals. This can help to avoid unnecessary processing of the EP sensor outputs. In a preferred embodiment, the mat determines the signal quality of the differential signal from pairs of EP sensors in turn and in a predetermined order until a minimum predetermined signal quality is achieved. If, once a predetermined series of EP sensor pairs has been tested and the minimum signal quality has not been achieved, the mat can move onto combinations of EP sensors pairs as described below, or could indicate to the user to change their position of engagement with the mat.

In preferred embodiments, the outputs of two or more EP sensors at each user interaction region can be combined so as to minimise the effect of noise and allow the system to cope with sub-optimal placement of the user's hands or feet on the mat (which could, for example, be due to a medical condition afflicting the user). An average of the signals of a plurality of EP sensors could be calculated for use as the EP signal from the respective user interaction region. Preferably the average is a weighted mean to which the contribution having the greatest weight is from an EP sensor positioned such that it engages with thenar eminence or hypothenar eminence (in the case of a hand) or the heel of the foot (in the case of the foot). The weighting applied to these EP sensors which typically yield the best ECG signal is preferably at least 50% more than that of any other sensor.

Generally the EP sensor pairs that provide a differential signal from which the ECG is identified comprise two sensors of the same type: for example, in the case of hands if the first sensor of the pair is a sensor under the ball of the thumb (thenar eminence) of the left hand then the second sensor of the pair would be a sensor under the ball of the thumb of the right hand. However, this need not be the case and the differential signal could be obtained from an EP sensor from one part of the left hand (e.g. under the ball of the thumb) and an EP sensor engaged with a different part of the right hand (e.g. the hypothenar eminence).

A mat configured in accordance with the present invention can reliably capture a good quality ECG of more than 10 seconds duration within around 60 seconds. Within the capture period, the EP sensor pairs from which the ECG signal is captured could change in order to maintain a clean ECG.

Preferably the mat further comprises one or more additional EP sensors 103, 104 that are provided as thin discs connected to the mat by wired or wireless means. These sensors can be used in various ways. Firstly, these sensors can be readily used to provide Lead II and III inputs to the mat: the user could place one or more of the free EP sensors underneath their feet (e.g. they could stand on them), or the EP sensors 103, 104 could be provided with a strap (e.g. a velcro strap) or other attaching means to attach them to the ankles or feet of the user. Secondly, the sensors could be used by patients that have trouble engaging with the mat due to problems placing their hands roughly flat on the mat surface over sensors 101, 102: the free sensors could be simply held by the user or strapped to their hand (e.g. by means of a velcro strap) such that the sensor is directed to one of the preferred fleshy parts of their hand (i.e. the thenar eminence or the hypothenar eminence). Input means to the mat can allow the user or a medical professional to indicate to the mat which part of the body the EP sensors are engaged with so as to allow the mat to properly process the acquired ECG.

The present invention is not limited to the detection of electrocardiographic (ECG) signals and could also be used for the detection of electromyographic (EMG) signals, i.e. the acquisition of electrical activity due to skeletal muscles. If camera 131 is present, it can be used to direct the user to place their limb in the optimal position for acquisition of the EMG signals of interest. For example, the control unit 112 could be configured to monitor the position of a user's forearm relative to sensors of the mat and direct by means of screen 126 the optimal placement of the forearm over those sensors in order to acquire a wrist muscle EMG. This can be achieved by causing a user to contract the particular muscle of interest, acquiring the EMG of that muscle or muscle group and comparing the characteristics of the EMG with a set of characteristics stored at data store 118. If the characteristics of the EMG suggest that the user's body part is not optimally positioned, the device can indicate to the user to reposition their body part. The device preferably indicates to the user the optimal position they should adopt.

By the provision of multiple sensors, the mat could be configured to acquire both ECG and EMG signals simultaneously. A system that incorporates the EP Sensors with an active feedback loop may have benefits in systems not necessarily for medical usages, such as sports and gym equipment.

Understanding the muscle EMG coupled with a cardiac ECG might allow an exercise programme to be controlled for the limited exercising of specific muscle groups as analysed by the EMG, and with positive feedback being given to the subject by means of the camera and screen.

EP sensors are typically much smaller than human hands or feet. Multiple EP sensors can therefore be allocated for each human limb. Through the use of statistical averaging over the sensors allocated to a limb (e.g. hand or foot) a cleaner signal can be acquired and in a shorted space of time.

Electrocardiograph device 105 may be configured to be foldable along folds 201 as illustrated in figure 2. This can have two benefits. Firstly, as shown in figure 2(b), the mat can then be stored more compactly. Secondly, as shown in figure 2(c), the mat can be folded into a three-dimensional object: for example, a tubular object of triangular cross-section as shown in figure (c), or a tubular object of rectangular cross-section if the mat were to have three folds 201. In figure 2(c), the folds of the mat are configured such that the surfaces of the mat comprising the EP sensors 202, 203 are presented to a user when the mat is rested on a third surface 204. Such a folding arrangement might allow the mat to be used more comfortably when an ECG signal is acquired from a pair of hands or any other body part.

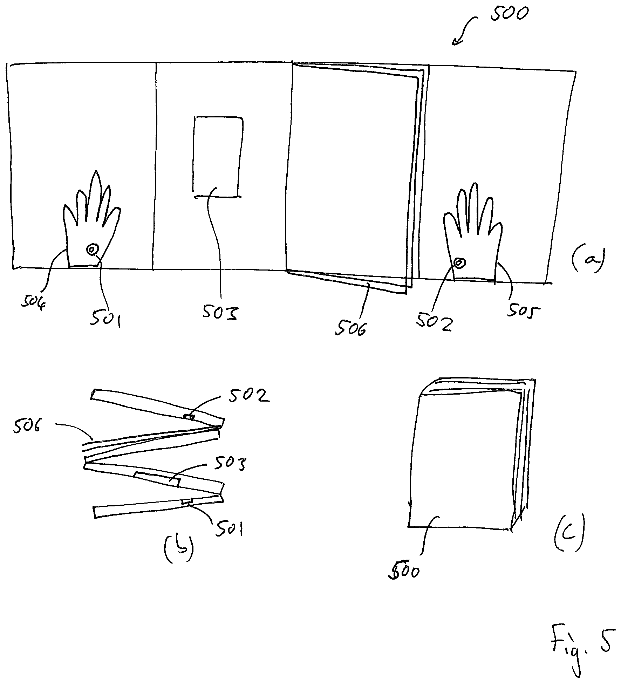

A further embodiment of the present invention is shown in figure 5, in which electrocardiograph device 500 is provided in the form of a book having EP sensors 501, 502 on the inside front and back covers. Indicia 504, 505 can be provided to indicate to a user how to arrange their hands relative to the EP sensors. The book could comprise a processor (not shown) and a touchscreen 503. The book comprises one or more pages 506 that preferably explain to the user how to use the device. Figure 5(a) shows the device when opened out flat for use, figure 5(b) shows the device in a partially-folded position, and figure 5(c) shows the device when fully folded up as a book. This embodiment provides the electrocardiograph device in a convenient form that is particularly suitable for use in the home or office, and allows the ECG to be readily stored in a form that is not obviously a medical device.

In an embodiment of the present invention, mat 105 could take the form of a sleeping mat, which could be used for an infant. Placing a patient on the sleeping mat would allow an ECG to be acquired by means of the EP sensors embedded in the surface of the mat. Such a sleeping mat is advantageous for two reasons. Firstly, the ECG itself can be used to ensure the safety of an infant patient since problems with the infant's heartbeat can be immediately alerted to the parent or carer. Secondly, the ECG can be used in a novel manner to determine the position in which an infant is sleeping and hence determine whether the infant is sleeping in a safe position.

This second use of an ECG acquired by a mat configured in accordance with the present invention relies on the fact that an ECG acquired by EP sensors varies in dependence on the position of the EP sensors on the patient's body. Thus, the ECG acquired from a patient sleeping on their back will be different from the ECG acquired from a patient sleeping on their front. By configuring control unit 112 to process the ECG of a patient in dependence on a set of stored characteristics indicative of various sleeping positions, the system can be configured to determine which position the patient is sleeping in. The control unit may be configured to display and/or sound an alarm or send a message via a communication means such as wifi, Bluetooth, etc. when an infant patient is found to be sleeping in an unsafe position, preferably by means of a baby monitor so as to avoid waking the infant.

A mat can be configured to determine the position of a subject placed upon it through analysis of the waveforms generated from the sensor pairs. Using the theory of Einthoven's triangle it is possible to determine which parts of the body a pair of sensors are likely to be engaged with since the shape of the waveform varies in dependence with the parts of the body from which electrical signals are captured. Thus the differential signal from a pair of sensors can be cross-referenced by its QRS complex, shape, magnitude, and polarity, with stored parameters describing the waveforms observed for leads I,II, and III.

Lead I looks horizontally from the left side of the heart. Lead II looks at the heart from the left leg. Lead III looks at the heart from the right leg, lead AVF (derived from the left leg and right arm leads) look from below towards the heart. It is further possible to work out if the sensors are acting in an anterior mode, with the subject lying face down upon them, or in a posterior mode, with the patient lying face up, with their back upon the sensors.

Once the patients face up/face down orientation is established, it is then possible to calculate the rotation of the person with regards a reference plane of the mat. Assuming that the subject may not be lying exactly face down or face up, the usage of the V1-V6 leads may be employed to augment the limb leads (Leads I,II,III) to establish the upper torso position of the subject upon the mat.

Once the orientation of the subject is established in three dimensional space, it may be possible to identify the heart axis as well. This is achieved by averaging all electrical signals from the heart, thus indicating the direction of the average electrical depolarization as a vector. This vector is the heart axis and a change in the heart axis or an extreme deviation can be an indication of pathology. To determine the heart axis the extremity leads only (not V1-V6) are averaged. The heart's axis can be estimated by focusing on leads I, II, and AVF. A left heart axis is present when the QRS in lead I is positive and negative in II and AVF. (between -30 and -90 degrees, A right heart axis is present when lead I is negative and AVF positive. (between +90 and + 180, an extreme heart axis is present when both I and AVF are negative, however this is a rare finding.

The ECG signals acquired by the EP sensors of the mat are analysed at control unit 112 after digital encoding at analogue to digital converter 116 and transmission over link 110. By comparing the signal acquired at one EP sensor with that acquired at another EP sensor according to a common time base, the various leads of an ECG system can be determined and derived as is known in the art. The control unit 112 may be able to run any form of digital analysis on the acquired ECG data, such as algorithms for arrhythmia detection.

The ECG signal could be cleaned up by analogue electronics at the input of AtoD converter 116 and/or its feedback loop 113, or by means of digital electronics at the output of the AtoD converter or at the control unit 112. The AtoD converter preferably samples at between 1 and 5kHz.

As described above, a QRS detection algorithm is used to identify the basic features of the ECG signal from the differential output of a pair of EP sensors. Further algorithms can then be used to identify various characteristics of the ECG and it is possible to analysis an ECG signal from more than one starting point. For example, a P wave algorithm analyses the signal by detecting the presence of the P wave, whereas an R-R algorithm analyses the signal by detecting the R-R interval in the ECG signal. Generally, either type of algorithm can be used in the analysis of an ECG.

It is advantageous if the mat is configured to utilise one of the types of algorithm in dependence on an initial parse of the ECG signal (which could be performed by the QRS detection algorithm). If the initial signal shows a high amplitude signal has been recorded then there is a high probability that the system should be able to detect a strong P wave should it exist. Preferably, if the amplitude of the QRS complex in the detected signal is greater than the noise floor by a predetermined value (e.g. equivalent to 0.8 * S wave amplitude in V1 + R wave amplitude in V5 < 3.5 mV for a conventional ECG), then a P wave algorithm is used. Otherwise an R-R interval algorithm is used.

Suitable P wave detection algorithms include those described in "Detection of P and T-waves in Electrocardiogram" by S. S. Mehta and N. S. Lingayat, Proceedings of the World Congress on Engineering and Computer Science 2008, San Francisco, USA and "Characteristic wave detection in ECG signal using morphological transform" by Yan Sun et al., BMC Cardiovascular Disorders Vol. 28, Issue 5, 2005.

A suitable R-R interval detection algorithm is described in "A simple method to detect atrial fibrillation using RR intervals" by Lian et al, American Journal of Cardiology, Vol. 107, Issue 10, 2011.

Suitable QRS detection algorithms are described in "Quantitative investigation of QRS detection rules using the MIT/BIH arrhythmia database", IEEE Transactions on Biomedical Engineering, Vol. BME-33, No. 12, 1986.

It can be further advantageous to indicate to the user when the ECG captured by the differential output of a pair of ECG sensors (i.e. a one-lead ECG) is of poor quality. In particular, it is proposed that in preferred embodiments the mat is configured to indicate to the user (e.g. by means of screen 126) that the captured signal is of poor quality and that the user should therefore move to using a three-lead ECG configuration by utilising an additional mat coupled to the first mat. For example, if the first mat is configured to receive inputs from a user's hands, the mat could indicate to the user to additionally engage with a mat configured to receive inputs from one or more of the user's feet. By combining the outputs of the respective ECG sensors at one/both of the mats or at an external or remote control unit connected to the mats (such as a laptop) a three-lead ECG can be calculated.

By directing the user to engage with the ECG monitoring system of the present invention in this manner, the system aims to ensure that the captured ECG is of sufficient quality to ensure that the sensitivity and specificity values of the P wave and R-R interval algorithms can be maintained, thus increasing the confidence factor of the system.

During both the signal acquisition phase and during the continuously returned signal monitoring phase, owing to the high frequency and the ability of the EP sensors to positively feedback coherent signals into the stage amplifier (typically part of the AtoD 116 and feedback loop 113), it is likely that other bio-information may be detected, such as the respiration rate of the subject, or the electrical signal of a particular muscle group.

It can also be advantageous to arrange that specific biomarkers other than the ECG are carried in the signals from the EP sensors and not removed by filtering. Thus not only the ECG signal may be encoded but also additional signals from different muscle groups (or other anatomical entities). If the control unit cannot itself process such additional signals, they can be stored by the control unit for later analysis.

At the commencement of any ECG reading delivered through the utilisation of the EP sensors, there will be a period of time before the system locks onto the ECG signal when the level of background noise is relatively high. This inhibits for a short period the identification of a clean ECG signal trace, or a signal trace that is of high enough quality to form the input data for a cleaning and refinement algorithm operating on the control unit.

There is therefore required a mechanism between an EP sensor and the control unit that allows the establishment of a clear to send/ready to send handshake. This may well be implemented in software such that the EP sensor output at analogue level may be encoded with a preceding marker bit that can be set such that, when the analogue side of the analogue to digital converter has found and isolated an ECG signal from the background noise, that it can set the marker bit as ready to send, thus allowing the analogue to digital converter to signal to the control unit that it is ready to send over link 110. This first marker bit may be acknowledged with a clear to send bit, which in turn is then acknowledged by the analogue to digital converter. Whereupon the streaming of data may commence. Such transmission mechanisms are known in the art from modems and connection orientated communications protocol such as TCP/IP.

Screen 126 may be used to give feedback to the subject with regards the duration that the subject should keep their limbs engaged with the surface. In such a manner if the control unit that received the data from the plurality of EP sensors and passed this to data to an algorithm to analyse, and subsequently the algorithm identified that the user had a known problem with their physiological data, then the system, through the screen could inform the subject to extend their testing period for a known period of time, such that the condition identified may be verified by taking a longer reading of physiological data over a longer period of time, thus reducing the potential for false positive analysis.

The present system can be utilised to take a quick ECG reading through limb placement over a multitude of EP sensors and lends itself to being able to provide a quick ECG analysis of current cardiac performance. Even over a time period of perhaps only 90 seconds it may well be possible to not only run an algorithm on the system to detect arrhythmia and other well documented cardiac conditions, but also to export the statistics of each cardiac or physiological parameter recorded, and then to calculate the average or median performance of each physiological cycle. Such data can be used in the control inputs of a medical scoring system, such as the Karnofsky, Zubrod, Apache III, or Geneva scoring systems.

Where specific physiological monitoring scoring systems exist for say cardiology, it may be possible to identify a risk stratification for the subject based on the output performance characteristics of their sinus rhythm with regards the time span that they were monitored by the EP sensors. In such a manner it may well be possible to extrapolate a whole series of performance indices, such as average QRS complex time. Such indices can be provided with the ECG traces themselves at the output 129 of the control unit, and/or at screen 126. These indices, coupled with any artefacts identified by the unique to subject start up sequence acquired by each EP sensor, can yield artefacts for further analysis which can be output from the control unit.

The data from EP sensors may be transmitted over link 110 in a high level format such as XML or HL7. Preferably timestamps are encoded into such a high level stream format, along with optionally an indication of location of the parts of the anatomy being monitored/the location of EP sensors on a subject's body. This may prove beneficial in the production of body mapped electrical systems activity and subsequent multi-dimensional imagery through the utilisation of a plurality of sensor, perhaps in the region of 120 EP sensors in a system to wrap and encompass the upper torso of the human body. Such a system could be incorporated into a shirt or other clothing offering.

The output of the source ECG signal that has been acquired from the limb placement upon the EP sensors, may well be output in a high level format such as HL7, XML, etc, in such a manner that the original source ECG trace may be available for analysis in conjunction with any events that caused arrhythmias to be identified already correlated to the source ECG trace. In a similar manner any identified other physiological parameters identified may also be included in the analysis.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features. In view of the foregoing description it will be evident to a person skilled in the art that various modifications may be made within the scope of the invention.

## Claims

1. An electrocardiographic device for sensing cardiac activity in a user at the user's hands comprising:
a pair of electrically insulating user interaction regions (301), each adapted for engagement with a hand of a user;
a first set of electric potential sensors (101, 102) provided at the user interaction regions and each configured to provide an electric potential sensor output signal;
a first set of indicia (504, 505) indicating a first position of a user's hands with respect to the first set of electric potential sensors; and
wherein each user interaction region includes movement sensing means (305) configured to provide a movement signal that, in use when a user's hands are in the first position, represents movement and/or vibration of the user's hands and
wherein the electrocardiographic device is configured to use each movement signal to filter out corresponding variations in the electric potential sensor output signal(s) from the electric potential sensors of the respective user interaction region; **characterised in that** the first set of sensors (101, 102) are arranged relative to the first set of indicia such that, in use when a user's hands are in the first position, an electric potential sensor is located under the thenar eminence and/or an electric potential sensor is located under the hypothenar eminence of each of the user's hands.

2. An electrocardiographic device as claimed in claim 1, wherein the indicia comprise a graphical pair of hands indicating the desired location of a user's hands relative to the sensors (101, 102) and/or indentations on the surface of the device indicating the desired location of a user's hands relative to the sensors (101, 102).

3. An electrocardiographic device as claimed in any preceding claim, wherein each user interaction region (301) comprises a layer of shape memory foam (302) for engagement with a user's hand, the first set of electric potential sensors being provided at the shape memory foam.

4. An electrocardiographic device as claimed in any preceding claim, wherein each user interaction region is supported on a floating plate (307) elastically coupled to the bulk of the electrocardiographic device.

5. An electrocardiographic device as claimed in claim 4, wherein both user interaction regions are supported at the same floating plate.

6. An electrocardiographic device as claimed in claims 4 or 5, wherein each floating plate (307) is elastically coupled to the bulk of the electrocardiographic device by one or more of: a spring optionally with a damper, a rubber or plastic element, and elastic foam (306).

7. An electrocardiographic device as claimed in any preceding claim, wherein the movement sensors (305) are one or more of an accelerometer, a strain gauge, a microphone, a pressure sensor, and an electric potential sensor arranged such that, in use, the electric potential sensor does not engage with the user.

8. An electrocardiographic device as claimed in any preceding claim, further comprising a second set of indicia indicating a second position of a user's hands with respect to the first set of electric potential sensors; wherein the first set of sensors (101, 102) are arranged relative to the second set of indicia such that, in use when a user's' hands are in the second position, at least one sensor is located under the medial side of the hypothenar eminence of each of the user's hands.

9. An electrocardiographic device as claimed in any preceding claim, wherein the electrocardiographic device comprises at least two electric potential sensors at each user interaction region.

10. An electrocardiographic device as claimed in any preceding claim, wherein each electric potential sensor comprises a conducting electrode coated with a thin insulating layer such that, in use, at least the thin insulating layer separates the user from the conducting electrode.

11. An electrocardiographic device as claimed in any preceding claim, wherein each electric potential sensor is provided within but electrically insulated from a conductive patch such that, in use, the user is in electrical contact with the conductive patch, the conductive patch being connected to an electrical ground of the electrocardiographic device.

12. An electrocardiographic device as claimed in any preceding claim, further comprising a display screen (126) operable to display one or more of: a graphic illustrating a user's hand engaging with a user interaction region in the first position; a message directing the user to engage with a user interaction region in a position other than the first position; an electrocardiograph acquired by the first set of electric potential sensors.

13. An electrocardiographic device as claimed in any preceding claim, wherein the electrocardiographic device is configured to be foldable along one or more folds (201) such that, when folded, the device is operable to present at least one of the first set of electric potential sensors on each of two outward-facing surfaces of the device, the two outward-facing surfaces being separated by at least one fold.

14. An electrocardiographic device as claimed in any preceding claim, wherein the electrocardiographic device is provided in the form of a book having one or more pages, at least one of the first set of electric potential sensors (501, 502) being provided at the front and rear inside covers of the book.

15. An electrocardiographic device as claimed in claim 14, wherein the pages of the book provide instructions for use of the electrocardiographic device.

## Patentansprüche

1. Elektrokardiographische Vorrichtung zum Erfassen einer Herzaktivität in einem Benutzer an den Händen des Benutzers, umfassend:
ein Paar elektrisch isolierender Benutzerinteraktionsbereiche (301), die jeweils zur Ineingriffnahme durch eine Hand eines Benutzers ausgelegt sind;
einen ersten Satz von Sensoren (101, 102) für elektrisches Potential, die in den Benutzerinteraktionsbereichen bereitgestellt sind und jeweils konfiguriert sind, ein Sensorausgabesignal über ein elektrisches Potential bereitzustellen;
einen ersten Satz von Zeichen (504, 505), die eine erste Position der Hände eines Benutzers in Bezug auf den ersten Satz von Sensoren für elektrisches Potential anzeigen; und
wobei jeder Benutzerinteraktionsbereich Bewegungserfassungsmittel (305) beinhaltet, die konfiguriert sind, ein Bewegungssignal bereitzustellen, das während des Gebrauchs, wenn sich die Hände eines Benutzers in der ersten Position befinden, eine Bewegung und/oder Vibration der Hände des Benutzers darstellt; und
wobei die elektrokardiographische Vorrichtung konfiguriert ist, jedes Bewegungssignal zu verwenden, um entsprechende Variationen der Sensorausgabesignale über das elektrische Potential von den Sensoren für elektrisches Potential des jeweiligen Benutzerinteraktionsbereichs herauszufiltern; **dadurch gekennzeichnet, dass** der erste Satz von Sensoren (101, 102) in Bezug auf den ersten Satz von Zeichen so angeordnet ist, dass sich während des Gebrauchs, wenn sich die Hände eines Benutzers in der ersten Position befinden, ein Sensor für elektrisches Potential unter dem Daumenballen befindet und/oder sich ein Sensor unter dem Kleinfingerballen von jeder der Hände des Benutzers befindet.

2. Elektrokardiographische Vorrichtung nach Anspruch 1, wobei die Zeichen ein grafisches Händepaar, das die gewünschte Lage einer Hand eines Benutzers in Bezug auf die Sensoren (101, 102) anzeigt, und/oder Markierungen auf der Fläche der Vorrichtung, die die gewünschte Lage einer Hand eines Benutzers in Bezug auf die Sensoren (101, 102) anzeigen, umfasst.

3. Elektrokardiographische Vorrichtung nach einem der vorhergehenden Ansprüche, wobei jeder Benutzerinteraktionsbereich (301) eine Schicht aus Formgedächtnisschaumstoff (302) zur Ineingriffnahme durch eine Hand eines Benutzers umfasst, wobei der erste Satz von Sensoren für elektrisches Potential am Formgedächtnisschaumstoff bereitgestellt ist.

4. Elektrokardiographische Vorrichtung nach einem der vorhergehenden Ansprüche, wobei jeder Benutzerinteraktionsbereich auf einer beweglichen Platte (307) getragen wird, die mit dem Großteil der elektrokardiographischen Vorrichtung elastisch gekoppelt ist.

5. Elektrokardiographische Vorrichtung nach Anspruch 4, wobei beide Benutzerinteraktionsbereiche an derselben beweglichen Platte getragen werden.

6. Elektrokardiographische Vorrichtung nach Anspruch 4 oder 5, wobei jede bewegliche Platte (307) elastisch mit dem Großteil der elektrokardiographischen Vorrichtung gekoppelt ist, durch eines oder mehrere von: einer Feder, optional mit einem Dämpfer, einem Gummi- oder Kunststoffelement und elastischem Schaumstoff (306) .

7. Elektrokardiographische Vorrichtung nach einem der vorhergehenden Ansprüche, wobei es sich bei den Bewegungssensoren (305) um eines oder mehrere von einem Beschleunigungssensor, einem Dehnungsmessstreifen, einem Mikrofon und einem Sensor für elektrisches Potential, der so angeordnet ist, dass der Sensor für elektrisches Potential während des Gebrauchs nicht durch den Benutzer in Eingriff genommen wird, handelt.

8. Elektrokardiographische Vorrichtung nach einem der vorhergehenden Ansprüche, ferner umfassend einen zweiten Satz von Zeichen, die eine zweite Position einer Hand eines Benutzers in Bezug auf den ersten Satz von Sensoren für elektrisches Potential anzeigen; wobei der erste Satz von Sensoren (101, 102) in Bezug auf den zweiten Satz von Zeichen so angeordnet ist, dass sich während des Gebrauchs, wenn sich die Hände eines Benutzers in der zweiten Position befinden, mindestens ein Sensor unter der medialen Seite des Kleinfingerballens von jeder der Hände des Benutzers befindet.

9. Elektrokardiographische Vorrichtung nach einem der vorhergehenden Ansprüche, wobei die elektrokardiographische Vorrichtung mindestens zwei Sensoren für elektrisches Potential in jedem Benutzerinteraktionsbereich umfasst.

10. Elektrokardiographische Vorrichtung nach einem der vorhergehenden Ansprüche, wobei jeder Sensor für elektrisches Potential eine leitende Elektrode umfasst, die mit einer dünnen Isolierschicht überzogen ist, sodass während des Gebrauchs mindestens die dünne Isolierschicht den Benutzer von der leitenden Elektrode trennt.

11. Elektrokardiographische Vorrichtung nach einem der vorhergehenden Ansprüche, wobei jeder Sensor für elektrisches Potential innerhalb von, aber elektrisch isoliert von, einer leitenden Stelle bereitgestellt ist, sodass der Benutzer während des Gebrauchs in elektrischem Kontakt mit der leitenden Stelle ist, wobei die leitende Stelle mit einer elektrischen Masse der elektrokardiographischen Vorrichtung verbunden ist.

12. Elektrokardiographische Vorrichtung nach einem der vorhergehenden Ansprüche, ferner umfassend einen Anzeigebildschirm (126), der funktionsfähig ist, eine oder mehrere der Folgenden anzuzeigen: eine Grafik, die eine Hand eines Benutzers darstellt, die in der ersten Position in Eingriff mit einem Benutzerinteraktionsbereich steht; eine Nachricht, die den Benutzer anweist, einen Benutzerinteraktionsbereich in einer anderen Position als der ersten Position in Einriff zu nehmen; einen Elektrokardiograph, der durch den ersten Satz von Sensoren für elektrisches Potential erlangt wird.

13. Elektrokardiographische Vorrichtung nach einem der vorhergehenden Ansprüche, wobei die elektrokardiographische Vorrichtung so konfiguriert ist, dass sie entlang einer oder mehrerer Falzungen (201) faltbar ist, sodass die Vorrichtung, wenn sie gefaltet ist, funktionsfähig ist, mindestens einen von dem ersten Satz von Sensoren für elektrisches Potential auf jeder von zwei nach außen gerichteten Flächen der Vorrichtung darzustellen, wobei die zwei nach außen gerichteten Flächen durch mindestens eine Falzung getrennt sind.

14. Elektrokardiographische Vorrichtung nach einem der vorhergehenden Ansprüche, wobei die elektrokardiographische Vorrichtung in Form eines Buches bereitgestellt ist, das eine oder mehrere Seiten aufweist, wobei mindestens einer von dem ersten Satz von Sensoren (501, 502) für elektrisches Potential an dem vorderen und hinteren Inneneinband des Buches bereitgestellt ist.

15. Elektrokardiographische Vorrichtung nach Anspruch 14, wobei die Seiten des Buches Anweisungen zur Verwendung der elektrokardiographischen Vorrichtung bereitstellen.

## Revendications

1. Dispositif électrocardiographique pour la détection de l'activité cardiaque chez un utilisateur 2 en surpoids, les mains de l'utilisateur comprenant :
une paire de régions d'interaction utilisateur isolantes électriquement (301), chacune adaptée à l'engagement avec une main d'un utilisateur ;
un premier ensemble de capteurs de potentiel électrique (101, 102) fournis dans les régions d'interaction utilisateur et chacun configuré pour fournir un signal de sortie de capteur de potentiel électrique ;
un premier ensemble d'indices (504, 505) indiquant une première position des mains de l'utilisateur en ce qui concerne le premier ensemble de capteurs de potentiel électrique ; et
dans lequel chaque région d'interaction utilisateur comprend des moyens de détection de mouvement (305) configurés pour fournir un signal de mouvement qui, en cours d'utilisation lorsque les mains d'un utilisateur sont en première position, représente le mouvement et/ou la vibration des mains de l'utilisateur ; et
dans lequel le dispositif électrocardiographique est configuré pour utiliser chaque signal de mouvement pour filtrer les variations correspondantes dans le ou les signal(aux) de sortie du capteur de potentiel électrique à partir des capteurs de potentiel électrique de la région d'interaction utilisateur respective ; **caractérisé en ce que** le premier ensemble de capteurs (101, 102) est disposé par rapport au premier ensemble d'indices de sorte que, en cours d'utilisation lorsque les mains d'un utilisateur sont dans la première position, un capteur de potentiel électrique est situé sous l'éminence thénar et/ou un capteur de potentiel électrique est situé sous l'éminence hypothénar de chacune des mains de l'utilisateur.

2. Dispositif électrocardiographique selon la revendication 1, dans lequel les indices comprennent une paire graphique de mains indiquant l'emplacement souhaité des mains d'un utilisateur par rapport aux capteurs (101, 102) et/ou aux indentations à la surface du dispositif indiquant l'emplacement souhaité des mains d'un utilisateur par rapport aux capteurs (101, 102).

3. Dispositif électrocardiographique selon l'une quelconque des revendications précédentes, dans lequel chaque région d'interaction utilisateur (301) comprend une couche de mousse à mémoire de forme (302) pour l'engagement avec une main d'un utilisateur, le premier ensemble de capteurs de potentiel électrique étant fourni à la mousse à mémoire de forme.

4. Dispositif électrocardiographique selon l'une quelconque des revendications précédentes, dans lequel chaque région d'interaction utilisateur est prise en charge sur une plaque flottante (307) couplée de manière élastique à la majeure partie du dispositif électrocardiographique.

5. Dispositif électrocardiographique selon la revendication 4, dans lequel les deux régions d'interaction utilisateur sont prises en charge sur la même plaque flottante.

6. Dispositif électrocardiographique selon les revendications 4 ou 5, dans lequel chaque plaque flottante (307) est couplée de manière élastique à la majeure partie du dispositif électrocardiographique par un ou plusieurs de : un ressort facultativement avec un amortisseur, un élément en caoutchouc ou en plastique et une mousse élastique (306).

7. Dispositif électrocardiographique selon l'une quelconque des revendications précédentes, dans lequel les capteurs de mouvement (305) sont un ou plusieurs d'un accéléromètre, d'une jauge de contrainte, d'un microphone, d'un capteur de pression et d'un capteur de potentiel électrique disposé de sorte que, en cours d'utilisation, le capteur de potentiel électrique ne s'engage pas avec l'utilisateur.

8. Dispositif électrocardiographique selon l'une quelconque des revendications précédentes, comprenant en outre un deuxième ensemble d'indices indiquant une deuxième position des mains d'un utilisateur en ce qui concerne le premier ensemble de capteurs de potentiel électrique ; dans lequel le premier ensemble de capteurs (101, 102) est disposé par rapport au deuxième ensemble d'indices de sorte que, en cours d'utilisation lorsque des mains d'un utilisateur sont dans la deuxième position, au moins un capteur est situé sous le côté médial de l'éminence hypothénar de chacune des mains de l'utilisateur.

9. Dispositif électrocardiographique selon l'une quelconque des revendications précédentes, dans lequel le dispositif électrocardiographique comprend au moins deux capteurs de potentiel électrique dans chaque région d'interaction utilisateur.

10. Dispositif électrocardiographique selon l'une quelconque des revendications précédentes, dans lequel chaque capteur de potentiel électrique comprend une électrode conductrice revêtue d'une fine couche isolante telle que, en cours d'utilisation, au moins la fine couche isolante sépare l'utilisateur de l'électrode conductrice.

11. Dispositif électrocardiographique selon l'une quelconque des revendications précédentes, dans lequel chaque capteur de potentiel électrique y est fourni, mais isolé électriquement à partir d'un patch conducteur de sorte que, en cours d'utilisation, l'utilisateur est en contact électrique avec le patch conducteur, le patch conducteur étant connecté à une mise électrique à la terre du dispositif électrocardiographique.

12. Dispositif électrocardiographique selon l'une quelconque des revendications précédentes, comprenant en outre un écran d'affichage (126) utilisable pour afficher un ou plusieurs de : un graphique illustrant une main d'un utilisateur s'engageant avec une région d'interaction utilisateur dans la première position ; un message dirigeant l'utilisateur pour qu'il s'engage avec une région d'interaction utilisateur dans une position autre que la première position ; un électrocardiogramme acquis par le premier ensemble de capteurs électriques potentiels.

13. Dispositif électrocardiographique selon l'une quelconque des revendications précédentes, dans lequel le dispositif électrocardiographique est configuré pour être pliable le long d'un ou plusieurs plis (201) de sorte que, lorsqu'il est plié, le dispositif est utilisable pour présenter au moins l'un du premier ensemble de capteurs de potentiel électrique sur chacune des deux surfaces accessibles de l'extérieur du dispositif, les deux surfaces accessibles de l'extérieur étant séparées par au moins un pli.

14. Dispositif électrocardiographique selon l'une quelconque des revendications précédentes, dans lequel le dispositif électrocardiographique est fourni sous la forme d'un livre ayant une ou plusieurs pages, au moins l'un du premier ensemble de capteurs de potentiel électrique (501, 502) étant fourni sur les couvertures avant et arrière du livre.

15. Dispositif électrocardiographique selon la revendication 14, dans lequel les pages du livre fournissent des instructions d'utilisation du dispositif électrocardiographique.
